# EUROPÄISCHE PATENTSCHRIFT

(11) **EP 3 121 184 B1**
(45) Veröffentlichungstag und Bekanntmachung des Hinweises auf die Patenterteilung: **12.09.2018**
(21) Anmeldenummer: 16180045.3
(22) Anmeldetag: 19.07.2016
(51) Int. Cl.: B01J 31/22, B01J 31/24, C07C 67/38, C07F 17/02, C07F 9/572, C07F 9/655, C07F 9/6553, C07F 15/00, C07F 9/58, C07F 9/6506

(54) **BENZOLBASIERTE DIPHOSPHINLIGANDEN FÜR DIE ALKOXYCARBONYLIERUNG**
BENZENE-BASED DIPHOSPHINE LIGANDS FOR ALKOXYCARBONYLATION
LIGANDS DE DISPHOSPHINE À BASE DE BENZOL POUR L'ALKOXY-CARBONYLATION

(30) Priorität: 23.07.2015 DE 102015213918
(43) Veröffentlichungstag der Anmeldung: 25.01.2017
(73) Patentinhaber: Evonik Degussa GmbH, 45128 Essen (DE)
(72) Erfinder: DONG, Kaiwu, 236800 Bo Zhou (CN); NEUMANN, Helfried, 18055 Rostock (DE); JACKSTELL, Ralf, 27478 Cuxhaven Altenwalde (DE); BELLER, Matthias, 18211 Ostseebad Nienhagen (DE); FRANKE, Robert, 45772 Marl (DE); HESS, Dieter, 45770 Marl (DE); DYBALLA, Katrin Marie, 45657 Recklinghausen (DE); FRIDAG, Dirk, 45721 Haltern am See (DE); GEILEN, Frank, 45721 Haltern am See (DE)

(56) Entgegenhaltungen:
- WO-A1-2011/083305
- WO-A1-2011/152358
- H. BRUNNER ET AL: "Enantioselective catalyses 10. New chiral phosphanes derived from substituted quinolines", SYNTHESIS, 1. November 1997 (1997-11-01), Seiten 1309-1314, XP55321856,

## Beschreibung

Die Erfindung betrifft benzolbasierte Diphosphinverbindungen, Metallkomplexe dieser Verbindungen und deren Verwendung für die Alkoxycarbonylierung.

Die Alkoxycarbonylierung ethylenisch ungesättigter Verbindungen ist ein Prozess mit steigender Bedeutung. Unter einer Alkoxycarbonylierung versteht man die Umsetzung ethylenisch ungesättigter Verbindungen (Olefinen) mit Kohlenmonoxid und Alkoholen in Gegenwart eines Metall-Ligand-Komplexes zu den entsprechenden Estern. Üblicherweise wird als Metall Palladium eingesetzt. Das folgende Schema zeigt die allgemeine Reaktionsgleichung einer Alkoxycarbonylierung:

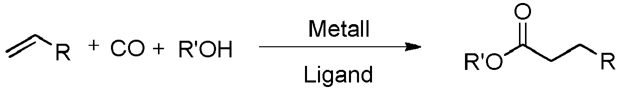

Unter den Alkoxycarbonylierungsreaktionen ist insbesondere die Reaktion von Ethen und Methanol zu 3-Methylpropionat (Ethen-Methoxycarbonylierung) von Bedeutung als Zwischenschritt für die Herstellung von Methylmethacrylat (S. G. Khokarale, E. J. Garcia-Suárez, J. Xiong, U. V. Mentzel, R. Fehrmann, A. Riisager, Catalysis Communications 2014, 44, 73-75). Die Ethen-Methoxycarbonylierung wird in Methanol als Lösemittel bei milden Bedingungen mit einem durch Phosphinliganden modifizierten Palladiumkatalysator durchgeführt.

Üblicherweise werden dabei zweizähnige Diphosphinverbindungen als Liganden eingesetzt. Ein sehr gutes katalytischen System wurde von Lucite - jetzt Mitsubishi Rayon - entwickelt und verwendet einen Liganden auf Basis von 1,2-Bis(di-tert-butylphosphinomethyl)benzol (DTBPMB) (W. Clegg, G. R. Eastham, M. R. J. Elsegood, R. P. Tooze, X. L. Wang, K. Whiston, Chem. Commun 1999, 1877-1878).

In WO 2011/083305 A1 werden bidentate Liganden beschrieben, welche einen Rest mit einem Stickstoffatom aufweisen, der einen pKb-Wert aufweist, der in einer wässrigen Lösung bei 18 °C in dem Bereich von 4 bis 14 liegt. Der Ligand kann zur Katalyse einer Carbonylierungsreaktion von ethylenisch ungesättigten Verbindungen eingesetzt werden.

Die vorliegende Erfindung hat die Aufgabe, neue Liganden für die Alkoxycarbonylierung bereitzustellen, mit denen sich höhere Ausbeuten an Estern erzielen lassen. Insbesondere sollen die erfindungsgemäßen Liganden für die Alkoxycarbonylierung von langkettigen ethylenisch ungesättigten Verbindungen, beispielsweise C₈-Olefinen, und von Gemischen ethylenisch ungesättigter Verbindungen geeignet sein. Auch die Anwesenheit von funktionellen Gruppen wird toleriert.

Diese Aufgabe wird gelöst durch benzolbasierte Diphosphinverbindungen, die an wenigstens einem Phosphoratom mit wenigstens einem Heteroarylrest substituiert sind. Diese Verbindungen eignen sich in besonderer Weise als zweizähnige Liganden für Palladiumkomplexe und führen zu erhöhten Ausbeuten bei der Alkoxycarbonylierung von einer Vielzahl unterschiedlicher ethylenisch ungesättigter Verbindungen. Die erfindungsgemäßen Liganden führen außerdem zu einer geringeren Bildung von Ethern als Nebenprodukt und verbessern die n/iso-Selektivität der Alkoxycarbonylierung.

Die erfindungsgemäßen Diphosphinverbindungen sind Verbindungen gemäß Formel (**I**) 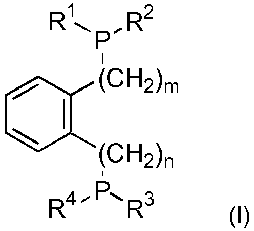 wobei
m und n unabhängig voneinander jeweils 0 oder 1 sind;
die Reste R¹ und R³ jeweils für einen -(C₃-C₂₀)-Heteroarylrest stehen;
und R² und R⁴ jeweils unabhängig voneinander ausgewählt sind aus -(C₁-C₁₂)-Alkyl und R¹, R², R³, R⁴, falls diese für -(C₁-C₁₂)-Alkyl oder -(C₃-C₂₀)-Heteroaryl stehen,
jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl,-COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl,-(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

In einer Ausführungsform handelt es sich bei den erfindungsgemäßen Diphosphinverbindungen um Verbindungen gemäß einer der Formeln (**II**) und (**III**) 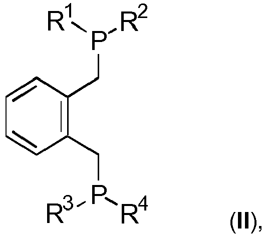 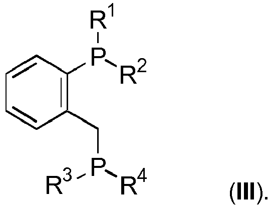 Dabei haben die Reste R¹, R², R³, R⁴ jeweils die oben genannte Bedeutung.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃₋C₁₂)-Cycloalkyl, -COO-(C₃-C₁₂)-Cycloalkyl, -CONH-(C₃-C₁₂)-Cycloalkyl, -CO-(C₃-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff Halogen umfasst insbesondere Fluor, Chlor, Brom und lod. Besonders bevorzugt sind Fluor und Chlor.

In einer Ausführungsform können die Reste R¹, R², R³, R⁴, falls diese für -(C₁-C₁₂)-Alkyl oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl,-S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein.

In einer Ausführungsform können die Reste R¹, R², R³, R⁴, falls diese für -(C₁-C₁₂)-Alkyl oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl,-(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C3-C₂₀)-Heteroaryl-O-(C₁-C₁₂)-Alkyl, substituiert sein.

In einer Ausführungsform können die Reste R¹, R², R³, R⁴, falls diese für -(C₁-C₁₂)-Alkyl oder -(C₃-C₂₀)-Heteroaryl stehen, jeweils unabhängig voneinander mit einem oder mehreren Substituenten ausgewählt aus -(C₁-C₁₂)-Alkyl, -O-(C₁-C₁₂)-Alkyl-(C₆-C₂₀)-Aryl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl substituiert sein.

In einer Ausführungsform können die Reste R¹, R², R³, R⁴, falls diese für -(C₁-C₁₂)-Alkyl oder -(C₃-C₂₀)-Heteroaryl stehen, jeweils unabhängig voneinander mit einem oder mehreren Substituenten ausgewählt aus -(C₁-C₁₂)-Alkyl und -(C₃-C₂₀)-Heteroaryl substituiert sein.

In einer Ausführungsform sind die Reste R¹, R², R³, R⁴ unsubstituiert, falls diese für -(C₁-C₁₂)-Alkyl oder -(C₃-C₂₀)-Heteroaryl stehen.

In einer Ausführungsform sind die Reste R¹, R³ jeweils unabhängig voneinander ausgewählt aus Heteroarylresten mit fünf bis zehn Ringatomen, vorzugsweise fünf oder sechs Ringatomen.

In einer Ausführungsform stellen die Reste R¹, R³ einen Heteroarylrest mit fünf Ringatomen dar.

In einer Ausführungsform sind die Reste R¹, R³ jeweils unabhängig voneinander ausgewählt aus Heteroarylresten mit sechs bis zehn Ringatomen.

In einer Ausführungsform stellen die Reste R¹, R³ einen Heteroarylrest mit sechs Ringatomen dar.

In einer Ausführungsform sind die Reste R¹, R³ ausgewählt aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl, wobei die genannten Heteroarylreste wie oben beschrieben substituiert sein können.

In einer Ausführungsform sind die Reste R¹, R³ ausgewählt aus Furyl, Thienyl, Pyrrolyl, Imidazolyl, Pyridyl, Pyrimidyl, Indolyl, wobei die genannten Heteroarylreste wie oben beschrieben substituiert sein können.

In einer Ausführungsform sind die Reste R¹, R³ ausgewählt aus 2-Furyl, 2-Thienyl, 2-Pyrrolyl, 2-Imidazolyl, 2-Pyridyl, 2-Pyrimidyl, 2-Indolyl, wobei die genannten Heteroarylreste wie oben beschrieben substituiert sein können.

In einer Ausführungsform sind die Reste R¹, R³ ausgewählt aus 2-Furyl, 2-Thienyl, N-Methyl-2-Pyrrolyl, N-Phenyl-2-Pyrrolyl, N-(2-Methoxyphenyl)-2-Pyrrolyl, 2-Pyrrolyl, N-Methyl-2-Imidazolyl, 2-Imidazolyl, 2-Pyridyl, 2-Pyrimidyl, N-Phenyl-2-Indolyl, 2-Indolyl, wobei die genannten Heteroarylreste nicht weiter substituiert sind.

Besonders bevorzugt stellen die Reste R¹, R³ Pyridyl dar, insbesondere 2-Pyridyl.

In einer Ausführungsform stellen R¹ und R³ einen Pyridylrest, bevorzugt 2-Pyridyl, dar und R² und R⁴ stehen für -(C₁-C₁₂)-Alkyl, wobei R¹, R², R³ und R⁴ jeweils wie oben beschrieben substituiert sein können.

In einer Ausführungsform sind die erfindungsgemäßen Diphosphinverbindungen ausgewählt aus einer der Formeln (**1**) und (**18**):

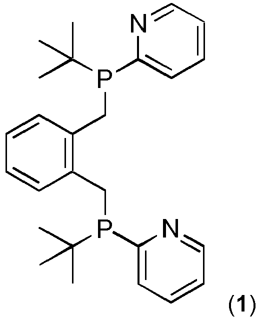

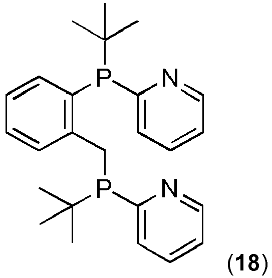

Die Erfindung betrifft weiterhin Komplexe umfassend Pd und eine erfindungsgemäße Diphosphinverbindung. Bei diesen Komplexen dient die erfindungsgemäße Diphosphinverbindung als zweizähniger Ligand für das Metallatom. Die Komplexe dienen beispielsweise als Katalysatoren für die Alkoxycarbonylierung. Mit den erfindungsgemäßen Komplexen lassen sich hohe Ausbeuten bei der Alkoxycarbonylierung einer Vielzahl unterschiedlicher ethylenisch ungesättigter Verbindungen erzielen.

Die erfindungsgemäßen Komplexe können außerdem weitere Liganden umfassen, die an das Metallatom koordinieren. Dabei handelt es sich beispielsweise um ethylenisch ungesättigte Verbindungen oder Anionen. Geeignete zusätzliche Liganden sind beispielsweise Styrol, Maleinimide (z.B. N-Methylmaleinimid), 1-4-Naphthochinon, Acetat-Anionen, Trifluoroacetat-Anionen oder Chloridanionen.

Die Erfindung betrifft weiterhin die Verwendung einer erfindungsgemäßen Diphosphinverbindung zur Katalyse einer Alkoxycarbonylierungsreaktion. Dabei kann die erfindungsgemäße Verbindung insbesondere als erfindungsgemäßer Metallkomplex eingesetzt werden.

Die Erfindung betrifft außerdem ein Verfahren umfassend die Verfahrensschritte:
a) Vorlegen einer ethylenisch ungesättigten Verbindung;
b) Zugabe einer erfindungsgemäßen Diphosphinverbindung und einer Verbindung, welche Pd umfasst,
   oder Zugabe eines erfindungsgemäßen Komplexes umfassend Pd und eine erfindungsgemäße Diphosphinverbindung;
c) Zugabe eines Alkohols;
d) Zuführen von CO;
e) Erwärmen des Reaktionsgemisches, wobei die ethylenisch ungesättigte Verbindung zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform umfasst die ethylenisch ungesättigte Verbindung 2 bis 30 Kohlenstoffatome, bevorzugt 6 bis 22 Kohlenstoffatome, besonders bevorzugt 8 bis 12 Kohlenstoffatome, am meisten bevorzugt 8 Kohlenstoffatome.

Die ethylenisch ungesättigten Verbindungen können zusätzlich zu der einen oder mehreren Doppelbindungen weitere funktionelle Gruppen enthalten. Vorzugsweise umfasst die ethylenisch ungesättigte Verbindung eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten. Dabei umfasst die ethylenisch ungesättigte Verbindung vorzugsweise insgesamt 2 bis 30 Kohlenstoffatome, bevorzugt 2 bis 22 Kohlenstoffatome, besonders bevorzugt 2 bis 12 Kohlenstoffatome.

In einer Ausführungsform umfasst die ethylenisch ungesättigte Verbindung keine weiteren funktionellen Gruppen außer Kohlenstoff-Kohlenstoff-Doppelbindungen.

In einer besonders bevorzugten Ausführungsform handelt es sich bei der ethylenisch ungesättigten Verbindung um ein unfunktionalisiertes Alken mit mindestens einer Doppelbindung und 2 bis 30 Kohlenstoffatomen, bevorzugt 6 bis 22 Kohlenstoffatomen, weiter bevorzugt 8 bis 12 Kohlenstoffatomen, am meisten bevorzugt 8 Kohlenstoffatomen.

Geeignete ethylenisch ungesättigte Verbindungen sind beispielsweise:
Ethen;
Propen;
C4-Olefine, wie 1-Buten, cis-2-Buten, trans-2-Buten, Gemisch von cis- und trans-2-Buten, Isobuten, 1,3-Butadien; Raffinat I bis III, Crack-C4
C5-Olefine, wie 1-Penten, 2-Penten, 2-Methyl-1-buten, 2-Methyl-2-buten, 2-Methyl-1,3-butadien (Isopren), 1,3-Pentadien;
C6-Olefine, wie Tetramethylethylen, 1,3-Hexadien, 1,3-Cyclohexadien;
C7-Olefine, wie 1-Methylcyclohexen, 2,4-Heptadien, Norbonadien;
C8-Olefine, wie 1-Octen, 2-Octen, Cycloocten, Di-n-buten, Di-iso-buten, 1,5-Cyclooctadien, 1,7-Octadien;
C9-Olefine, wie Tripropen;
C10-Olefine, wie Dicylcopentadien;
Undecene;
Dodecene;
interne C14-Olefine;
interne C15- bis C18-Olefine;
lineare oder verzweigte, cyclische, acyclische oder teilweise cyclische, interne C15- bis C30-Olefine;
Triisobuten, Tri-n-buten;
Terpene, wie Limonen, Geraniol, Farnesol, Pinen, Myrcen, Carvon, 3-Caren;
mehrfach ungesättigte Verbindungen mit 18 Kohlenstoffatomen, wie Linolsäure oder Linolensäure;
Ester ungesättigter Carbonsäuren, wie Vinylester von Essig- oder Propansäure, Alkylester ungesättigter Carbonsäuren, Methyl- oder Ethylester von Acrylsäure oder Methacrylsäure, Ölsäureester, Ölsäure Methyl- oder Ethylester, Ester der Linol- oder Linolensäure; Vinylverbindungen, wie Vinylacetat, Vinylcyclohexen, Styrol, alpha-Methylstyrol, 2-Isopropenylnaphthalin;
2-Methyl-2-pentenal, Methyl-3-Pentenoat, Methacrylanhydrid.

In einer Variante des Verfahrens ist die ethylenisch ungesättigte Verbindung ausgewählt aus Propen, 1-Buten, cis- und/oder trans-2-Buten, oder Mischungen davon.

In einer Variante des Verfahrens ist die ethylenisch ungesättigte Verbindung ausgewählt aus 1-Penten, cis- und/oder trans-2-Penten, 2-Methyl-1-buten, 2-Methyl-2-buten, 3-Methyl-1-buten, oder Mischungen davon.

In einer bevorzugten Ausführungsform ist die ethylenisch ungesättigte Verbindung ausgewählt aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, n-Octen, 1-Octen, 2-Octen, oder Mischungen davon.

Die ethylenisch ungesättigte Verbindung kann zusätzlich ausgewählt sein unter 1,7-Octadien, Cycloocten, 10-Undecensäure-Methylester, 1-Methyl-cyclohexen, 5-Hexennitril, 6-Chlor-1-hexen, Vinyltriethylsilan, Carvon, Isopropenylbenzol, 4-Chlor-Isopropenylbenzol, 4-Fluor-Isopropenylbenzol, 2-Methyl-Isopropenylbenzol, 2-Isopropenylnaphthalen, 1,1-Diphenylethylen, 1,3-Diisopropenylbenzol, 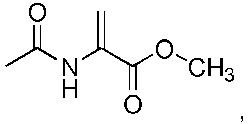 Tetramethylethylen, N-Vinylphthalimid, 2,3,4,5,6-Pentafluorstyrol, 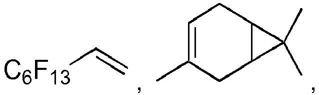 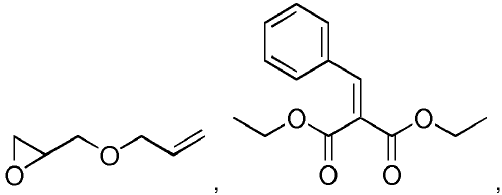 trans-2-Butensäure-Ethylester.

In einer Variante wird ein Gemisch ethylenisch ungesättigter Verbindungen eingesetzt. Als Gemisch wird im Sinne dieser Erfindung eine Zusammensetzung bezeichnet, die wenigsten zwei verschiedene ethylenisch ungesättigte Verbindungen enthält, wobei der Anteil jeder einzelnen ethylenisch ungesättigten Verbindungen vorzugsweise wenigstens 5 Gewichts-% bezogen auf das Gesamtgewicht des Gemisches beträgt.

Vorzugsweise wird ein Gemisch ethylenisch ungesättigter Verbindungen mit jeweils 2 bis 30 Kohlenstoffatomen, bevorzugt 4 bis 22 Kohlenstoffatomen, besonders bevorzugt 6 bis 12 Kohlenstoffatomen, am meisten bevorzugt 8 bis 10 Kohlenstoffatomen eingesetzt.

Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren *n*-Butenen, Isobutan und *n*-Butan nach Abtrennung von Isobuten aus Raffinat I. Raffinat III ist ein Teil der beim Naphthacracken entstehenden C₄-Fraktion und besteht im Wesentlichen aus den isomeren *n*-Butenen und *n*-Butan.

Ein weiteres geeignetes Gemisch ist Di-n-Buten, auch bezeichnet als Dibuten, DNB oder DnB. Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder teilweise umgesetzt werden. Neben dem dimeren Di-n-Buten, entstehen in der Regel auch höhere Oligomere (Tributen C12, Tetrabuten C16), die nach der Reaktion destillativ abgetrennt werden. Diese können ebenfalls als Edukte eingesetzt werden.

In einer bevorzugten Variante wird ein Gemisch umfassend iso-Buten, 1-Buten, cis- und trans-2-Buten eingesetzt. Vorzugsweise umfasst das Gemisch 1-Buten, cis- und trans-2-Buten.

Die erfindungsgemäße Alkoxycarbonylierung wird durch den erfindungsgemäßen Pd-Komplex katalysiert. Der Pd-Komplex kann dabei in Verfahrensschritt b) entweder als präformierter Komplex umfassend Pd und den erfindungsgemäßen Phosphinliganden zugegeben werden, oder *in situ* aus einer Verbindung, welche Pd umfasst, und dem freien Phosphinliganden gebildet werden. Dabei wird die Verbindung, welche Pd umfasst, auch als Katalysatorvorstufe bezeichnet.

In dem Fall, dass der Katalysator *in situ* gebildet wird, kann der Ligand im Überschuss zugegeben werden, so dass im Reaktionsgemisch auch ungebundener Ligand vorliegt.

Auch im Falle des Komplexes, der gleich zu Beginn zugesetzt wird, kann auch weiterer Ligand zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂].

Vorzugsweise handelt es sich bei der Verbindung, welche Pd umfasst, um PdCl₂, Pd(acac)₂ oder Pd(OAc)₂. Besonders geeignet ist PdCl₂.

Der Alkohol im Verfahrensschritt c) kann verzweigt oder linear sein, cyclisch, alicyclisch, teilweise zyklisch oder aliphatisch und stellt insbesondere ein C₁-bis C₃₀-Alkanol dar. Es können Monoalkohole oder Polyalkohole verwendet werden.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl- , Amino-, Ether-, Thioether-, Aryl-, Heteroaryl- oder Silylgruppen, und/oder Halogensubstituenten enthalten.

In einer Ausführungsform umfasst der Alkohol keine weiteren funktionellen Gruppen außer Hydroxylgruppen.

Der Alkohol kann ungesättigte und aromatische Gruppen enthalten. Vorzugsweise handelt es sich jedoch um einen aliphatischen Alkohol.

Als aliphatischer Alkohol wird im Rahmen dieser Erfindung ein Alkohol bezeichnet, der keine aromatischen Gruppen umfasst, also beispielsweise ein Alkanol, Alkenol oder Alkinol.

In einer Ausführungsform handelt es sich bei dem Alkohol um ein Alkanol mit einer oder mehrerer Hydroxylgruppen und 1 bis 30 Kohlenstoffatomen, bevorzugt 1 bis 22 Kohlenstoffatomen, besonders bevorzugt 1 bis 12 Kohlenstoffatomen, am meisten bevorzugt 1 bis 6 Kohlenstoffatomen.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, Iso-Propanol, Iso-Butanol, tert-Butanol, 1-Butanol, 2-Butanol, 1-Pentanol, 2-Pentanol, 3-Pentanol, 1-Hexanol, Cyclohexanol, Phenol, 2-Ethylhexanol, Isononanol, 2-Propylheptanol.

In einer bevorzugten Variante ist der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: Cyclohexan-1,2-diol, 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol, 1,1,1-Tri(hydroxymethyl)ethan, Brenzkatechin, Resorcin und Hydroxyhydrochinon.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus: Methanol, Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um die ethylenisch ungesättigte Verbindung zu einem Ester umzusetzen.

Das molare Verhältnis von der in Schritt a) vorgelegten ethylenisch ungesättigten Verbindung zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu der in Schritt a) vorgelegten ethylenisch ungesättigten Verbindung beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis der erfindungsgemäßen Diphosphinverbindung zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Vorzugsweise wird das Verfahren unter Zusatz einer Säure durchgeführt. In einer Variante umfasst das Verfahren deshalb zusätzlich den Schritt c'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol-%, bevorzugt 0,4 bis 15 mol-%, besonders bevorzugt 0,5 bis 5 mol-%, am meisten bevorzugt 0,6 bis 3 mol-%, bezogen auf die Stoffmenge der in Schritt a) eingesetzten ethylenisch ungesättigten Verbindung.

### Beschreibung der Abbildungen

- Figur 1:: Methoxycarbonylierung von Di-n-Buten mit Ligand **1** bei 120 °C und 40 bar.
- Figur 2:: Methoxycarbonylierung von Di-n-Buten mit Ligand **1** bei 100 °C und 12 bar.

### Beispiele

Die folgenden Beispiele veranschaulichen die Erfindung.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Darstellung von Chlor-2-pyridyl-tert-butylphosphin (Vorstufe A)

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

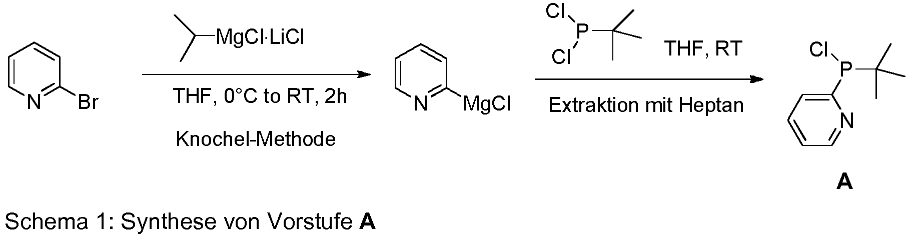

In einen 50 ml-Rundkolben mit Magnetrührer und Septum werden unter Argon 8,07 ml einer 1,3 M Isopropylmagnesium-chloridlösung (Knochel-Reagenz) gegeben und auf-15 °C gekühlt. Danach werden zügig 953.5 µl (10 mmol) 2-Bromopyridin zugetropft. Die Lösung wird sofort gelb. Man lässt auf-10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen. Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt kann die Grignardlösung zu einer Lösung von 1,748 g (11 mmol) Dichloro-tert-butylphosphin in 10 ml THF, die vorher auf -15 °C gekühlt worden ist, mit einer Spritzenpumpe langsam zugetropft werden. Wichtig ist, dass die Dichloro-tert-butylphosphinlösung gekühlt wird. Bei Raumtemperatur würde man erhebliche Mengen an Dipyridyl-tert-butylphosphin erhalten. Es entsteht anfangs eine klare gelbe Lösung, die dann trübe wird. Man lässt auf Raumtemperatur aufwärmen und über Nacht Rühren. Laut GCMS hat sich viel Produkt gebildet. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4,6 mbar, 120 °C Ölbad und 98 °C Übergangstemperatur kann das Produkt destilliert werden. Man erhält 1,08 g eines farblosen Öls. (50%).
Analytische Daten: ¹H NMR (300 MHz, C₆D₆): δ 8,36 (m, 1H, Py), 7,67 (m, 1H, Py), 7,03-6,93 (m, 1H, Py), 6,55-6,46 (m, 1H, Py), 1,07 (d, J = 13,3 Hz, 9H, t-Bu).
¹³C NMR (75 MHz, C₆D₆): δ 162,9, 162,6, 148,8, 135,5, 125,8, 125,7, 122,8, 35,3, 34,8, 25,9 und 25,8.
³¹P NMR (121 MHz, C₆D₆) δ 97,9.
MS (EI) *m*:*z* (relative Intensität) 201 (M⁺,2), 147(32), 145 (100), 109 (17), 78 (8), 57,1 (17).

### Darstellung von Verbindung 1 (α,α'-Bis(2-pyridyl(t-butyl)phosphino)o-xylen)

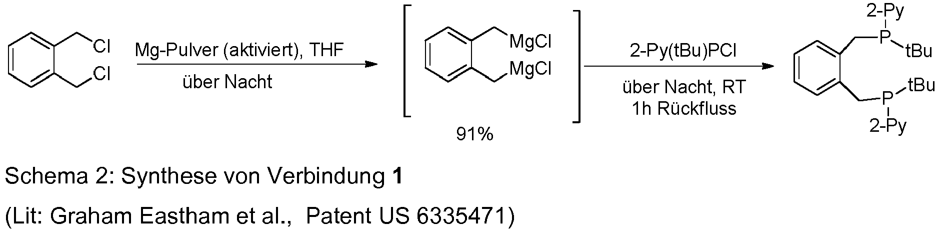

675 mg (27,8 mmol, 4 eq) Mg-Pulver werden in der Glovebox in einem 250 ml-Rundkolben mit Stickstoffhahn und Magnetrührkern eingewogen und mit einem Septum verschlossen. Man legt an den Rundkolben Hochvakuum (ca. 5x10⁻² mbar) an und erwärmt für 45 Minuten auf 90 °C. Nach Abkühlen auf Raumtemperatur werden 2 Körnchen Iod zugegeben und in 20 ml THF gelöst. Man rührt die Suspension ca. 10 Minuten bis die gelbe Farbe des Iods verschwunden ist. Nach Absetzen des Magnesiumpulvers wird die trübe THF-Lösung dekantiert und das aktivierte Magnesiumpulver 2 Mal mit 1-2 ml THF gewaschen. Dann werden erneut 20 mL frisches THF zugegeben. Bei Raumtemperatur wird eine Lösung von 1,21 g (6,9 mmol) α,α'-Dichloro-o-xylene in 70 ml THF mit der Spritzenpumpe langsam zugetropft. Die THF-Lösung verfärbt sich langsam dunkel. Am nächsten Tag wird die THF-Suspension von dem nicht umgesetzten Magnesiumpulver filtriert und der Gehalt an Grignardverbindung wird wie folgt bestimmt:
1 ml Grignardlösung wird in einer gesättigten wässrigen Lösung von NH₄Cl gequencht und mit Ether extrahiert. Nach Trocknen über Na₂SO₄ wird von der Etherlösung ein GC aufgenommen. Man sieht qualitativ, dass ausschließlich o-Xylen entstanden ist.

### Quantitative Bestimmung von dem Gehalt der Grignardlösung:

1 ml Grignardlösung wird mit 2 ml 0,1 M HCL gequenched und die überschüssige Säure mit 0,1 M NaOH titriert. Als Indikator ist eine wässrige 0,04%ige Bromkresollösung geeignet. Farbumschlag geht von gelb nach blau. Es sind 0,74 ml an 0.1 M NaOH verbraucht worden. 2mL-0,74 ml = 1,26 ml, das entspricht 0,126 mmol Grignardverbindung. Da ein Digrignard vorliegt, ist die Grignardlösung 0,063 M. Das sind über 90% Ausbeute.

In einem 250mL Dreihalskolben mit Rückflusskühler und Magnetrührer werden unter Argon 1,8 g (8,66 mmol) Chlorphosphin (2-Py(tBu)PCI) in 10 ml THF gelöst und auf-60 °C gekühlt. Dann werden 55 ml der oben bestimmten Grignardlösung (0,063 M, 3,46 mmol) langsam bei dieser Temperatur mit der Spritzenpumpe zugetropft. Die Lösung bleibt zunächst klar und wird dann intensiv gelb. Nach 1,5 Stunden wird die Lösung trüb. Man lässt über Nacht auf Raumtemperatur aufwärmen und man erhält eine klare gelbe Lösung. Zur Vervollständigung der Reaktion erhitzt man 1 Stunde unter Rückfluss. Nach Abkühlen gibt man 1 ml H₂O dazu und die Lösung entfärbt sich und wird milchig trüb. Nach Entfernen von THF im Hochvakuum erhält man einen zähen, hellgelben Feststoff. Man gibt 10 ml Wasser und 10 ml Ether dazu und erhält zwei homogene klare Phasen, die sich gut trennen lassen. Die wäßrige Phase wird zweimal mit Ether extrahiert. Nach Trocknen der organischen Phase mit Na₂SO₄ wird der Ether im Hochvakuum entfernt und man erhält einen zähen fast farblosen Feststoff. Dieser wird in 5 mL MeOH unter Erwärmen auf dem Wasserbad gelöst und über Celite filtriert. Bei -28 °C erhält man über Nacht 772 mg Produkt in Form von weißen Kristallen. (51%). Aus der Mutterlösung konnten nach Einengen nochmals 100 mg isoliert werden. Die Gesamtausbeute beträgt 57,6 %.
¹H NMR (300 MHz, C₆D₆): δ 8,58 (m, 2H, Py), 7,31-7,30 (m, 2H, Benzol), 7,30-7,22 (m, 2H, Py), 6,85-6,77 (m, 2H, Py), 6,73 (m, 2H, Benzol), 6,57-6,50 (m, 2H, py), 4,33(dd, J = 13,3 und 4,3 Hz, 2H, CH₂), 3,72-3,62 (m, 2H, CH₂), 121(d, J = 11,8 Hz, 18H, tBu),
¹³C NMR (75 MHz, C₆D₆): δ 161,3, 161,1, 149,6, 137,8, 137,7, 134,5, 133,3, 132,7, 131,4, 131,3, 125,7, 122,9, 30,7, 30,5, 28,2, 28,0, 26,5, 26,4, 26,2, und 26,1.
³¹P NMR (121 MHz, C₆D₆) δ 8,8, EA berechnet für C₂₆H₃₄N₂P₂: C, 71,54; H, 7,85; N, 6,56; P,14,35, gefunden: C, 71,21; H, 7,55; N, 6,56; P, 14,35.

### Darstellung von Verbindung 18 (1-(2-pyridyl(t-butyl)phosphino)-2-(2-pyridyl(t-butyl) phosphinomethyl)benzol)

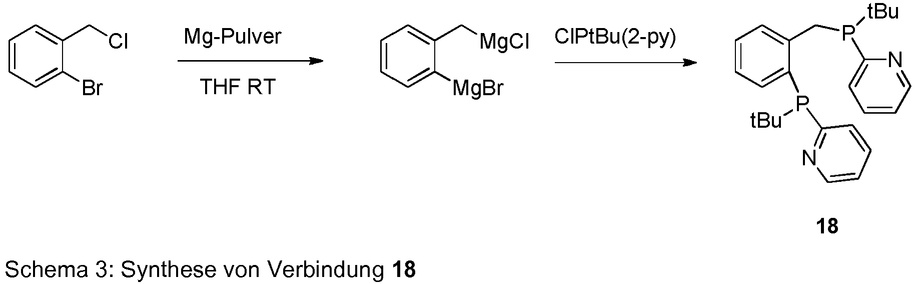

675 mg (27,8 mmol, 4 eq) Mg-Pulver werden in der Glovebox in einen 250 ml-Rundkolben mit Stickstoffhahn und Magnetrührkern eingewogen und mit einem Septum verschlossen. Man legt an den Rundkoben Hochvakuum (ca. 5x10⁻²) an und erwärmt ihn für 45 Minuten auf 90 °C. Nach Abkühlen auf Raumtemperatur werden 2 Körnchen Iod zugegeben und in 20 ml THF gelöst. Man rührt die Suspension ca. 10 Minuten bis die gelbe Farbe des Iods verschwunden ist. Nach Absetzen des Magnesiumpulvers wird die trübe THF-Lösung dekantiert und das aktivierte Magnesiumpulver zweimal mit 1-2 ml THF gewaschen. Dann werden erneut 20 ml frisches THF zugegeben. Bei Raumtemperatur wird eine Lösung von 920,7 µl (6,9 mmol) 2-Bromobenzylchlorid in 70 ml THF mit der Spritzenpumpe langsam zugetropft. Die THF-Lösung wird trübe und leicht grünlich. Am nächsten Tag liegt eine grünliche, milchige Suspension vor. Nach Absetzen lassen des überschüssigen Mg-pulvers wird die Suspension dekantiert und der Gehalt an Grignard-Verbindung bestimmt:
1 ml Grignardlösung wird mit 2 ml 0,1M HCl gequencht und die überschüssige Säure mit 0,1 M NaOH titriert. Als Indikator ist eine wässrige 0,04%ige Bromkresollösung geeignet. Farbumschlag geht von gelb nach blau. Es sind 0,4 mL an 0,1 M NaOH verbraucht worden. 2mL-0.4mL = 1,6mL, das entspricht 0,15 mmol Grignardverbindung. Da ein Di-grignard vorliegt ist die Grignardlösung 0,075 M.

In einem 250 ml-Dreihalskolben mit Rückflusskühler werden unter Argon 2,64 g (13,12 mol, 2,5 eq) Chlorphosphin (2-Py(tBu)PCl) in 15 ml THF gelöst und auf -60 °C gekühlt. Dann werden 70 mL der milchig-grünlichen Grignardlösung (0,075M, 5,25 mmol) langsam bei dieser Temperatur mit der Spritzenpumpe zugetropft. Während des dreistündigen Zutropfens ist keine wesentliche Veränderung der Reaktionslösung sichtbar. Man lässt über Nacht auf Raumtemperatur aufwärmen und erhält eine klare, dunkelgelbe Lösung. Zur Vervollständigung der Reaktion erhitzt man 2 Stunden unter Reflux. Nach Abkühlen gibt man 1 ml H₂O dazu und entfernt das THF im Hochvakuum. Man erhält einen zähen gelblichen Feststoff. Danach werden 20 ml Wasser und 30 ml Ether dazugegeben und man erhält eine inhomogene Lösung mit schlechter Phasentrennung. Durch Zugabe von abs. Methanol kann die Phasentrennung beschleunigt werden. Die wässrige Phase wird zweimal mit Ether extrahiert. Die vereinigte Etherphase ist klar und gelb. Nach Abziehen des Ethers erhält man 2,1 g eines hellorangen Rohprodukts. Dieses lässt sich nicht aus Methanol kristallisieren. Zur Reinigung des Produktes wird das Phosphin in das entsprechende Boranaddukt überführt:
Man löst das Rohprodukt in 15 ml THF auf und gibt 11,55ml (2,3 eq) 1 M Boran/THF-Komplex auf einmal dazu. Man lässt einen Tag bei Raumtemperatur rühren und chromatographiert das Boranaddukt mit einer Combi-Flash Apparatur (Ethylacetat/Heptan = 1:10). Man erhält 1,18 g (52%) eines weißen, porösen Feststoffes. Aus den Spektren geht hervor, dass 2 Diastereomere vorliegen müssen:
¹H NMR (400 MHz, CDCl₃): δ 8,76 und 8,73 (m, 2H, arom), 8,10 (m, 1H, arom), 7,92-7,62 (m, 3H, arom), 7,40 (m, 2H, arom), 7,31 (m, 1H, arom), 4,22-3,92 (m, 2H, CH₂), 1,51 (d, J = 14,5 Hz, tBu, 3H), 1,45 (d, J = 14,5 Hz, tBu, 6H), 1,23 (d, J = 14,5 Hz, 3H, tBu), 1,22 (d, J = 14,5 Hz, tBu, 6H).
³¹P NMR (161 MHz, CDCl₃) δ 37,71 (d, breit, J = 52,8 Hz), 36,52 (d, breit, J = 52,9 Hz), 33,65 (s, breit), 31,90 (s, breit).

Um das freie Phosphin zu erhalten, wird das Boranaddukt in 20 ml Morpholin gelöst und 4 Stunden auf 50 °C erwärmt. Danach entfernt man das Morpholin im Hochvakuum und chromatographiert den Rückstand unter Argon. (Ethylacetat/Heptan = 1:2). Das Produkt läuft auf der Säule vorne weg und kann so leicht isoliert werden. Das Lösungsmittel wird wieder entfernt und man erhält 1,1 g (98%) eines farblosen zähen Öles. Man sieht im Spektrum wieder zwei Diastereomere in einem Verhältnis von 1:2.
¹H NMR (300 MHz, C₆D₆): δ 8,65, 8,57 und 8,51 (m, 2H, arom), 7,58 und 7,51-7,33 (m, 4H, arom), 7,01 und 7,02-6,76 (m, 4H, arom), 6,63-6,45 (m, 2H, arom), 4,84, 4,47, 4,23 und 3,80 (m, 2H, CH₂), 1,53 (d, J = 12,8 Hz, 3H, tBu), 1,51 (d, J = 12,8 Hz, 6H, tBu*)*, 1,27-1,15 (m,9H, tBu).
¹³C NMR (75 MHz, C₆D₆): δ 167,2, 167,0, 149,7, 149,2, 149,0, 148,9, 137,1, 136,7, 136,3, 134,2, 134,1, 133,2, 132,6, 130,2, 130,1, 130,0, 129,6, 129,5, 129,2, 128,8, 125,6, 122,7, 122,5, 120,8, 120,6 (arom), 33,2, 33,0, 32,1, 31,4, 31,2 (q,tBu), 29,2, 28,9, 28,4, 28,2, 28,1, 27,9 (tBu), 27,6, 27,3 (CH₂), 22,9, 14,2.
³¹P NMR (121 MHz, C₆D₆) δ 17,90 (d, J = 29,2 Hz), 16,13 (d, J = 21,9 Hz), -0,59 (d, J = 21,9Hz), -0,73 (d, J = 29,2 Hz).

Elementaranalyse berechnet für C₂₅H₃₂N₂P₂: C, 71.07; H, 7.63; N, 6.63; P,14.66. Gefunden: C, 71.15; H, 8.20; N, 6.63; P,14.94.

### Liganden

In den nachfolgenden Versuchen zur Alkoxycarbonylierung werden die folgenden Liganden eingesetzt:
Ligand **1**, erfindungsgemäßes Beispiel 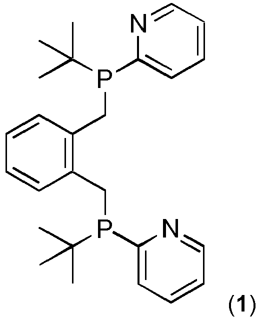
Ligand **18**, erfindungsgemäßes Beispiel 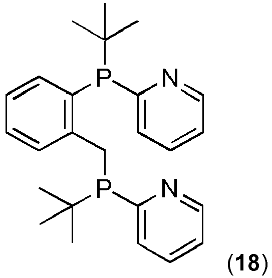
Ligand **3**, 1,2-Bis(di-tert-butylphosphinomethyl)benzol (DTBPMB), Vergleichsbeispiel

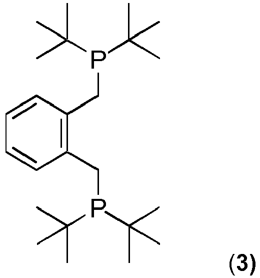

### Hochdruckexperimente

### Einsatzstoffe:

Di-n-Buten wurde auch wie folgt bezeichnet: Dibuten, DNB oder DnB.

Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder Teilweise umgesetzt werden. Neben dem dimeren Di-n-Buten, entstehen in der Regel auch höhere Oligomere (Tributen C12, Tetrabuten C16), die nach der Reaktion destillativ abgetrennt werden.

Ein industriell praktiziertes Verfahren zur Oligomerisierung von C4-Olefinen ist der sogenannte "OCTOL-Prozess".

Innerhalb der Patentliteratur beschreibt beispielsweise DE102008007081A1 eine auf dem OCTOL-Prozess beruhende Oligomerisierung. EP1029839A1 befasst sich mit der Fraktionierung der in dem OCTOL-Prozess entstehenden C8-Olefine.

Technisches Di-n-Buten besteht in der Regel zu 5 bis 30 % aus n-Octenen, 45 bis 75 % aus 3-Methylheptenen und zu 10 bis 35 % aus 3,4-Dimethylhexenen. Bevorzuge Ströme enthalten 10 bis 20 % n-Octene, 55 bis 65 % 3-Methylheptene und 15 bis 25 % 3,4-Dimethylhexene.

Para-Toluolsulfonsäure wurde wie folgt abgekürzt: pTSA, PTSA oder p-TSA. PTSA bezeichnet in diesem Text immer das para-Toluolsulfonsäure-Monohydrat.

### Allgemeine Vorschrift zur Durchführung der Hochdruckexperimente

### Allgemeine Versuchsbeschreibung für Reaktionen im Batchversuch:

Die entsprechenden Mengen an Substrat, Palladiumsalz, Säure und Alkohol werden unter Argon und Magnetrührung in einem 50 ml-Schlenkgefäß vermischt.

Ein 100 ml Stahlautoklav der Firma Parr versehen mit einem Gaseinlass und einem Gasauslassventil, einem digitalem Druckaufnehmer, einem Temperaturfühler und einem Kugelhahn sowie einer eingebauten Kapillare zur Probennahme wird dreimal mittels Vakuum und Argonspülung von Sauerstoff befreit. Anschließend wird die Reaktionslösung aus dem Schlenkgefäß mittels einer Kapillare in den Autoklaven im Argongegenstrom durch den Kugelhahn befüllt. Anschließend wird entweder bei Raumtemperatur die entsprechende Menge an CO aufgepresst und dann auf Reaktionstemperatur hochgeheizt (Reaktionen, die nicht unter konstantem Druck gefahren werden) oder es wird erst auf Reaktionstemperatur hochgeheizt und dann wird das CO über eine Bürette, die mittels eines Druckminderers mit dem Autoklaven verbunden ist, aufgepresst. Diese Bürette wird dann noch auf ca. 100 bar mit CO befüllt und liefert während der Reaktion das benötigte CO bei einem konstanten Druck nach. Diese Bürette hat ein Totvolumen von ca. 30 ml und ist mit einem digitalen Druckaufnehmer versehen. Dann wird die Reaktion bei der benötigten Temperatur die entsprechende Zeit unter Rührung durchgeführt. Hierbei werden mittels einer Software (Specview der Firma SpecView Corporation) und einem Prozesscontroller der Firma Parr 4870 sowie einem 4875 Powercontroler Daten über den Druckverlauf im Autoklaven und in der Gasbürette aufgezeichnet. Aus diesen werden Exceltabellen generiert, aus denen später Diagramme erstellt werden, die Gasverbräuche und damit Umsätze über die Zeit darstellen. Falls benötigt, werden über die Kapillare über die GC Proben gesammelt und analysiert. Hierzu wird vor der Reaktion eine geeignete exakte Menge (2-10 ml) Isooctan als interner Standard mit in das Schlenkgefäß gegeben. Diese geben auch Auskunft über den Reaktionsverlauf. Am Ende der Reaktion wird der Autoklav auf Raumtemperatur heruntergekühlt, der Druck vorsichtig abgelassen, falls nötig Isooctan als interner Standard hinzugefügt und eine GC-Analyse bzw. bei neuen Produkten auch eine GC MS Analyse durchgeführt.

### Allgemeine Versuchsvorschrift für Autoklavenversuche in Glasvials:

Es wird ein 300ml Parrreaktor verwendet. Diesem angepasst ist ein selbstgefertigter Aluminiumblock entsprechender Dimension welcher zu Heizen mittels handelsüblicher Magnetrührer z.B. der Firma Heidolph geeignet ist. Für das Innere des Autoklaven wurde eine runde Metallplatte der Stärke von ca. 1,5 cm angefertigt die 6 Bohrungen enthält, die dem Außendurchmesser der Glasvials entsprechen. Diesen Glasvials angepasst werden sie mit kleinen Magnetrührern bestückt. Diese Glasvials werden mit Schraubkappen und geeigneten Septen versehen und mit einer in der Glasbläserei angefertigten Spezialapparatur unter Argon mit den entsprechenden Reaktanden, Lösungsmittel und Katalysatoren und Additiven befüllt. Hierzu werden 6 Gefäße gleichzeitig befüllt, dies ermöglicht die Durchführung von 6 Reaktionen bei gleicher Temperatur und gleichem Druck in einem Experiment. Dann werden diese Glasgefäße mit Schraubkappen und Septen geschlossen und es wird jeweils ein kleine Spritzenkanüle geeigneter Größe durch die Septen gestochen. Dies ermöglicht später in der Reaktion den Gasaustausch. Diese Vials werden nun in der Metallplatte platziert und diese wird unter Argon in den Autoklaven überführt. Der Autoklav wird mit CO gespült und bei Raumtemperatur mit dem vorgesehenen CO-Druck befüllt. Dann wird mittels dem Magnetrührer unter Magnetrührung auf Reaktionstemperatur erhitzt und die Reaktion die entsprechende Zeit durchgeführt. Anschließend wird auf Raumtemperatur heruntergekühlt und der Druck langsam abgelassen. Anschließend wird der Autoklav mit Stickstoff gespült. Die Vials werden dem Autoklaven entnommen und mit einer definierten Menge eines geeigneten Standards versetzt. Es erfolgt eine GC Analyse mit deren Ergebnissen Ausbeuten und Selektivitäten bestimmt wurden.

### Allgemeine Vorschrift für Versuche in den 12fach-Autoklaven (600 ml Parr-Autoklav):

In ausgeheizte Glasvials werden jeweils Di-n-Buten (DnB) und Methanol vorgelegt, mit einer Lösung aus Pd(acac)₂ (0,5 mg, 0,0016 mmol) und Ligand (0,0064 mmol) in 0,2 ml Methanol versetzt und H₂SO₄ (Lösung: 1 ml H₂SO₄ in 50 ml MeOH) zugegeben. Im Autoklaven werden die Ansätze zweimal mit 10 bar CO gespült, mit dem gewünschten Druck CO beladen und bei der gewünschten Temperatur 20 h gerührt. Nach Beendigung der Reaktion werden jeweils Isooctan (interner Standard) und 1 ml EtOAc zugegeben. Die organische Phase wird per GC analysiert.

Die Ausbeuten der Reaktionen werden mittels GC (Isooctan als interner Standard) bestimmt.

### Analytik

GC Analytik der Produkte aus Ethen: Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1. Retentionszeit von Methylpropionat : 6.158 min

GC Analytik der Produkte aus Tetramethylethen: Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1.
Retentionszeit für Tetramethylethylen und Produkte: 7.436 min
Retentionszeit für den Ether: 11.391 min
Retentionszeit für Methyl 3,4-dimethylpentanoat: 17.269 min

GC Analytik Di-n-Buten: Für die GC Analyse wird ein Chromatograph der Firma Agilent GC Agilent 7890A mit einer 30 m HP5-Säule verwendet. Temperaturprofil: 35 °C, 10 min; 10 °C/min zu 200 °C; das Injektionsvolumen beträgt 1 µl mit einem Split von 50:1.

Retentionszeiten für Di-n-Buten und Produkte: 10.784-13.502 min

Die aus dem Di-n-Buten gebildeten Ester werden im Folgenden als MINO bezeichnet (Methylisononanoat).

Retentionszeit für Etherprodukte unbekannter Isomerenverteilung: 15.312, 17.042, 17.244, 17.417 min

Retentionszeit für iso-C9-Ester 19.502-20.439 min (Hauptpeak: 19.990 min)

Retentionszeit für n-C9-Ester: 20.669, 20.730, 20.884, 21.266 min.

### Auswertung der Versuche

Für die Auswertung der katalytischen Experimente werden im Folgenden bestimmte Kennzahlen verwendet, die einen Vergleich der verschiedenen Katalysatorsysteme zulassen.

TON: Turnovernumber, definiert als mol Produkt pro mol Katalysatormetall ist ein Maß für die Produktivität des katalytischen Komplexes.

TOF: Turnoverfrequenz, definiert als TON pro Zeit für das Erreichen eines bestimmten Umsatzes, z.B. 50 %. Die TOF ist ein Maß für die Aktivität des katalytischen Systems.

Die im Folgenden angegebenen n-Selektivitäten beziehen sich auf den Anteil der endständigen Methoxycarbonylierung bezogen auf die Gesamtausbeute an Methoxycarbonylierungsprodukten.

Das n/iso-Verhältnis gibt das Verhältnis von endständig zu Estern umgesetzten Olefinen zu innenständig zu Estern umgesetzten Olefinen an.

### Methoxycarbonylierung von Ethen

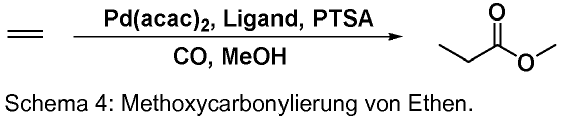

### a) Ligand 1

Ein 25 ml-Stahlautoklav wurde unter Argon mit PdCl₂ (2,53 mg, 0,04 mol%), **1** (24,9 mg, 0,16 mol%) und MeOH (5 ml) beladen. Dann wurde Ethen (1 g, 35,7 mmol) in den Autoklaven eingeleitet (Massenkontrolle über Waage). Der Autoklav wurde auf 80 °C aufgeheizt (Druck ca. 20 bar), dann wurden 30 bar CO aufgedrückt. Die Reaktion wurde bei 80 °C 20 Stunden durchgeführt.

Anschließend wurde der Autoklav auf Raumtemperatur abgekühlt und entspannt. Der Inhalt wurde in einen 50 ml-Schlenkkolben überführt und es wurden Isooctan (5 ml) als interner GC-Standard zugesetzt. Die Ausbeute wurde mittels GC-Analyse bestimmt. (Ausbeute: >99 %).

### b) Ligand 18

Ein 100 ml-Stahlautoklav wird mit Pd(acac)₂ (6,52 mg, 0,04 mol%) und Ligand **18** (36,1 mg, 0,16 mol%) und PTSA (61,1 mg, 0,6 mol%) und Methanol (20 ml) unter Argon befüllt. Dann werden 1,5 g, (53,6 mmol) Ethylen (3.5 von Linde AG) in den Autoklaven überführt. (Massekontrolle des Autoklaven). Nach dem Aufheizen auf eine Reaktionstemperatur von 80 °C (Druck ungefähr 10 bar), wird bei dieser Temperatur CO (30 bar) aufgepresst. Bei dieser Temperatur wird die Reaktion 20 Stunden durchgeführt. Dann wird der Autoklav auf Raumtemperatur heruntergekühlt und entspannt. Der Inhalt wird in ein 50 ml-Schlenkgefäß transferriert und Isooctan (interner Standard, 5,0 ml) wird zugesetzt. Die Ausbeute und Selektivität wurde mittels GC Analyse bestimmt. (Ausbeute: 92%).

Die erfindungsgemäßen Liganden **1** und **18** erzielen somit eine hohe Ausbeute bei der Methoxycarbonylierung von Ethen.

### Methoxycarbonylierung von Tetramethylethen in Gegenwart von PTSA

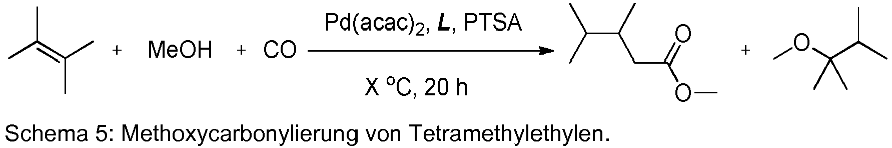

### a) Reaktionstemperatur: 100 °C

### (i) Ligand 3 (Vergleichsbeispiel)

Ein 25 ml-Schlenkgefäß wurde mit [Pd(acac)₂] (4,87 mg, 0,1 mol%), p-Toluolsulfonsäure (PTSA) (24,32 mg, 0,8 mol%) und MeOH (8 ml) befüllt. Ein 4 ml-Fläschchen wurde mit 3 (6,3 mg, 0,4 mol%) befüllt und ein Magnetrührstab hinzugegeben. Danach wurden 2 ml der klaren gelben Lösung und Tetramethylethen (478 µl, 4 mmol) mit einer Spritze injiziert. Das Fläschchen wurde auf einen Probenhalter gesetzt, der wiederum unter Argon-Atmosphäre in einen 300 ml-Parr-Autoklaven eingesetzt wurde. Nach dreimaligem Spülen des Autoklaven mit Stickstoff wurde der CO-Druck auf 40 bar eingestellt. Die Reaktion lief bei 100 °C 20 Stunden. Nach Abschluss der Reaktion wurde der Autoklav auf Raumtemperatur abgekühlt und vorsichtig entspannt. Isooctan (200 µl) wurde als interner GC-Standard zugesetzt. Ausbeute und Regioselektivität wurden mittels GC bestimmt. (Umsatz: 40%, keine Ester-Produktausbeute; Ether-Produktausbeute 38 %).

### (ii) Ligand 1

Ein 25 ml-Schlenkgefäß wurde mit [Pd(acac)₂] (4,87 mg, 0,1 mol%), p-Toluolsulfonsäure (PTSA) (24,32 mg, 0,8 mol%) und MeOH (8 ml) befüllt. Ein 4 mL-Fläschchen wurde mit **1** (7,0 mg, 0,4 mol%) befüllt und ein Magnetrührstab hinzugegeben. Danach wurden 2 ml der klaren gelben Lösung und Tetramethylethen (478 µl, 4 mmol) mit einer Spritze injiziert. Das Fläschchen wurde auf einen Probenhalter gesetzt, der wiederum unter Argon-Atmosphäre in einen 300 ml-Parr-Autoklaven eingesetzt wurde. Nach dreimaligem Spülen des Autoklaven mit Stickstoff wurde der CO-Druck auf 40 bar eingestellt. Die Reaktion lief bei 100 °C 20 Stunden. Nach Abschluss der Reaktion wurde der Autoklav auf Raumtemperatur abgekühlt und vorsichtig entspannt. Isooctan (200 µl) wurde als interner GC-Standard zugesetzt. Ausbeute und Regioselektivität wurden mittels GC bestimmt. (Umsatz: 82%, Ester-Produktausbeute: 60 %, Ether-Produktausbeute 20 %).

### b) Reaktionstemperatur: 120 °C

### (i) Ligand 3 (Vergleichsbeispiel)

Ein 25 ml-Schlenkgefäß wurde mit [Pd(acac)₂] (4,87 mg, 0,1 mol%), p-Toluolsulfonsäure (PTSA) (24,32 mg, 0,8 mol%) und MeOH (8 ml) befüllt. Ein 4 ml-Fläschchen wurde mit 3 (6,3 mg, 0,4 mol%) befüllt und ein Magnetrührstab hinzugegeben. Danach wurden 2 ml der klaren gelben Lösung und Tetramethylethen (478 µl, 4 mmol) mit einer Spritze injiziert. Das Fläschchen wurde auf einen Probenhalter gesetzt, der wiederum unter Argon-Atmosphäre in einen 300 ml-Parr-Autoklaven eingesetzt wurde. Nach dreimaligem Spülen des Autoklaven mit Stickstoff wurde der CO-Druck auf 40 bar eingestellt. Die Reaktion lief bei 120 °C 20 Stunden. Nach Abschluss der Reaktion wurde der Autoklav auf Raumtemperatur abgekühlt und vorsichtig entspannt. Isooctan (200 µl) wurde als interner GC-Standard zugesetzt. Ausbeute und Regioselektivität wurden mittels GC bestimmt. (Umsatz: 54%, keine Ester-Produktausbeute; Ether-Produktausbeute 52 %).

### (ii) Ligand 1

Ein 25 ml-Schlenkgefäß wurde mit [Pd(acac)₂] (4,87 mg, 0,1 mol%), p-Toluolsulfonsäure (PTSA) (24,32 mg, 0,8 mol%) und MeOH (8 ml) befüllt. Ein 4 ml-Fläschchen wurde mit **1** (7,0 mg, 0,4 mol%) befüllt und ein Magnetrührstab hinzugegeben. Danach wurden 2 ml der klaren gelben Lösung und Tetramethylethen (478 µL, 4 mmol) mit einer Spritze injiziert. Das Fläschchen wurde auf einen Probenhalter gesetzt, der wiederum unter Argon-Atmosphäre in einen 300 ml-Parr-Autoklaven eingesetzt wurde. Nach dreimaligem Spülen des Autoklaven mit Stickstoff wurde der CO-Druck auf 40 bar eingestellt. Die Reaktion lief bei 120 °C 20 Stunden. Nach Abschluss der Reaktion wurde der Autoklav auf Raumtemperatur abgekühlt und vorsichtig entspannt. Isooctan (200 µl) wurde als interner GC-Standard zugesetzt. Ausbeute und Regioselektivität wurden mittels GC bestimmt. (Umsatz: >99%, Ester-Produktausbeute: 98 %, keine Ether-Produktausbeute).

Wie den Ergebnissen entnommen werden kann, führt Ligand **1** führt bereits bei 100 °C und 40 bar CO-Druck zu einer höheren Ester-Produktausbeute als Vergleichsligand **3**. Zudem wird mit Ligand **1** ein geringerer Anteil von Nebenprodukt (Ether) gebildet.

### Methoxycarbonylierung von Tetramethylethylen in Gegenwart von Trifluormethansulfonsäure

Ein 100 ml-Stahlautoklav wird mit Pd(acac)₂ (4,87 mg, 0,04 mol%), Ligand 1 (28,0 mg, 0,16 mol%) und CF₃SO₂OH (72,1 mg, 1,28 mol%) befüllt .Dann werden unter Argon MeOH (20 ml), Isooctan (5 ml), und Tetramethylethylen (4,8 ml, 40 mmol) zugesetzt. Der Autoklav wird bei Raumtemperatur mit 40 bar CO befüllt. Die Reaktion wird 20 Stunden bei 120 °C durchgeführt. Während dieser Zeit werden mittels eines am Autoklaven installierten HPLC Ventils und einer internen Kapillare im Autoklaven, Proben zu verschiedenen Zeiten genommen. Diese werden mittels GC-Analyse untersucht und die Ausbeuten an den erwarteten Produkten bestimmt. Nachdem die Probennahmen beendet sind wird der Autoklav heruntergekühlt, entspannt und eine weitere finale GC-Probe entnommen und von dieser die Ausbeuten bestimmt. (98% Ausbeute an Methyl 3,4-dimethylpentanoat).

### Methoxycarbonylierung von di-n-Buten in Gegenwart von PTSA

Di-n-Buten ist ein Gemisch verschiedener Olefinisomeren mit 8 Kohlenstoffatomen. Herkunft und generelle Zusammensetzung wurden oben bereits erläutert. Das Verwendete DnB enthielt etwa 16 % (w/w) n-Octene, 65 % Methylheptene und 19 % Dimethylhexene.

Di-n-Buten wird mit Methanol zu Methylisononanoat (MINO) umgesetzt.

Bei der Alkoxycarbonylierung kommt es darauf an, möglichst gute Ausbeuten bei mittleren bis hohen Selektivitäten zum linearen Produkt zu erreichen und letztendlich mit kurzen Reaktionszeiten bis zur Endausbeute gute Raum-Zeit-Ausbeuten für ein technisches Verfahren zu erzielen.

**Ligand 1**: Ein 100 ml-Stahlautoklav wird unter Argon mit Pd(acac)₂ (5,85 mg, 0,04 mol%), 1 (33,5 mg, 0,16 mol%), MeOH (20 ml), 7,54 ml Di-n-Buten (48 mmol) und PTSA (para-Toluolsulfonsäure-Monohydrat) (54,7 mg, 0,6 mol%) befüllt. Dann werden auf den Autoklaven 40 bar CO bei Raumtemperatur aufgepresst. Die Reaktion wurde 20 Stunden bei 120 °C durchgeführt. Nach der Reaktion wird der Autoklav auf Raumtemperatur heruntergekühlt und der Druck abgelassen. Es werden 5 ml Isooctan als interner Standard zur Lösung gegeben. Die Ausbeute und Selektivität wurde mittels GC-Analyse bestimmt. (Ausbeute: 96%, n/iso: 73:27).

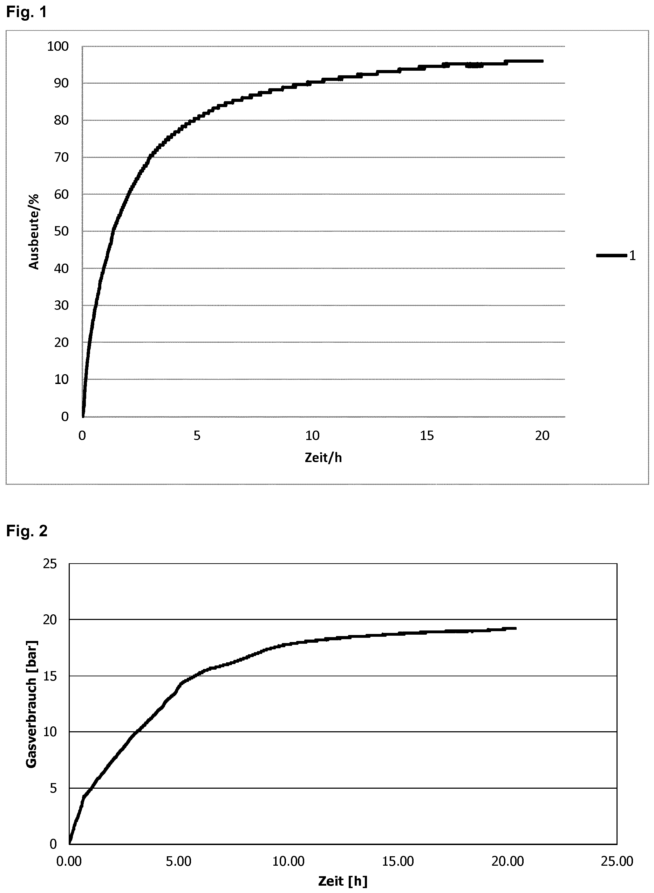

**Figur 1** zeigt den Ausbeute-Zeitverlauf dieser Reaktion.

**Ligand 3 (Vergleichsbeispiel)**: Ein 100 ml-Stahlautoklav wird unter Argon mit [Pd(acac)₂] (5,85 mg, 0,04 mol%), **3** (30,3 mg, 0,16 mol%) befüllt. Anschließend werden MeOH (30 ml) und Di-n-Buten (7,54 ml, 48 mmol) und PTSA (54,7 mg, 0,6 mol%) hinzugefügt. Der Autoklav wird bei Raumtemperatur mit 40 bar CO der Reinheit 4.7 befüllt und die Reaktion wird 20 Stunden bei 120 °C durchgeführt. Anschließend wird der Autoklav heruntergekühlt und der Druck langsam abgelassen. Der Autoklaveninhalt wird in ein Schlenkgefäß überführt. Es werden 5 ml Isooctan als interner Standard zugesetzt und die Ausbeute und Selektivität mittels GC Analyse bestimmt (60 % Ausbeute an MINO, *n*/*iso*: 93/7).

Diese Versuche zeigen, dass der erfindungsgemäße Ligand **1** eine höhere Ausbeute bei der Methoxycarbonylierung von di-n-Buten erzielt als der Vergleichsligand **3**.

### Methoxycarbonylierung von di-n-Buten in Gegenwart von Schwefelsäure

**Ligand 1**: In einem ausgeheizten Schlenkkolben werden jeweils 0,04 mol% Pd(acac)₂(15 mg) und 0,16 mol% 1 (105 mg) vorgelegt. Dann werden 12,4 ml (300 mmol) Methanol (techn.), 18,8 ml (120 mmol) Di-n-Buten und 32 µl (0,5 mol%) H₂SO₄ (98%ig) zugegeben und in einen 100 ml-Autoklaven überführt. Der Autoklav wird dann zweimal mit 10 bar CO gespült, mit 6 bar CO beladen und auf 100 °C erhitzt. Dann wird der Autoklav mittels Gasbürette mit 12 bar CO beladen und unter konstantem CO-Druck (12 bar) 20 h bei 100 °C gerührt. Nach Beendigung der Reaktion wird Isooctan (interner Standard) und 10 ml EtOAc zugegeben. Die organische Phase wird per GC analysiert. Die Ausbeute beträgt 91 %, die n-Selektivität 79 %.

Die Gasverbrauchskurve ist in **Figur 2** dargestellt.

Wie der Figur entnommen werden kann ist die Reaktion nach 20 Stunden weitgehend abgeschlossen. Die Gasaufnahme von ca. 20 bar korrespondiert mit der Ausbeute von 91 %.

### Methoxycarbonylierung von Ethylen und Di-n-Buten in Gegenwart von H₂

Die folgenden Versuche zeigen, dass bei Verwendung der erfindungsgemäßen Liganden die Ausbeute bei der Methoxycarbonylierung von Ethen oder Di-n-Buten durch eine Verunreinigung des CO-Gases mit H₂ nicht wesentlich beeinträchtigt wird. Das erfindungsgemäße Verfahren kann demnach auch in Gegenwart geringer Mengen von H₂ durchgeführt werden.

### a) Methoxycarbonylierung von Ethen mit Ligand 1

Ein 100 ml-Stahlautoklav wird mit Pd(acac)₂ (6,5 mg, 0,04 mol%), **1** (37,7 mg, 0,16 mol%), PTSA (61,1 mg, 0,6 mol%) und MeOH (20 ml) unter Argonatmossphäre befüllt. Dann wird Ethen (3.5 Linde) (1,5 g, 53,6 mmol) in den Autoklav überführt (Massekontrolle per Waage). H₂ (3 bar) und CO (30 bar) werden bei Raumtemperatur in den Autotklav eingetragen. Dann wird die Reaktion bei 80 °C für 20 Stunden durchgeführt. Anschließend wird der Autoklav heruntergekühlt und entspannt. Der Inhalt wird in ein 50 ml-Schlenkgefäß überführt und Isooctan (interner Standard, 3,0 ml) wird zugesetzt. Die Ausbeute wurde mittels GC Analyse bestimmt. (Ausbeute an Methylpropionat:99%).

### b) Methoxycarbonylierung von di-n-Buten mit Ligand 1

Ein 100 ml-Stahlautoklav wird unter Argon mit Pd(acac)₂ (5,8 mg, 0,04 mol%), Ligand **1** (33,5 mg, 0,16 mol%) und PTSA (54,7 mg, 0,6 mol%) befüllt. Dann werden MeOH (30 ml) und Di-n-Buten (7,54 mL, 48 mmol) unter Argon hinzugefügt. Der Autoklav wird bei Raumtemperatur mit H₂ (3 bar) und CO (40 bar) befüllt. Nach der Reaktion bei 120 °C und 20 Stunden wird der Autoklav heruntergekühlt und entspannt. Der Inhalt wird in ein 50 ml-Schlenkgefäß überführt und mit Isooctan (interner Standard, 8 ml) versetzt. Die Ausbeute und Regioselektivität wurde mittels GC Analyse bestimmt (Ausbeute: 94%, *n*/*iso*: 74/26).

### Methoxycarbonylierung verschiedener Olefine mit den Liganden 3 und 1

Reaktionsbedingungen für Ligand 3 (Vergleichsbeispiel): Ein 25 ml-Schlenkgefäß wird mit einer Stammlösung aus [Pd(acac)₂] (12,2 mg, 0,04 mol), **3** (63,1 mg, 0,16 mmol), PTSA (114 mg, 0,6 mmol) und MeOH (25 ml) unter Argon befüllt. Ein 4 ml-Glasvial versehen mit einem Magnetrührer, wird mit 2 mmol Olefin befüllt. Hierzu werden 1,25 ml der zuvor hergestellten Stammlösung mittels einer Spritze zugesetzt. Dieses Vial wird auf einer Metallplatte in einem 300 ml-Parr-Autoklaven unter Argon platziert. Der Autoklav wird dreimal mit CO gespült und anschließend werden 40 bar CO aufgepreßt. Dann wird unter Magnetrührung die Reaktion 20 h bei 120 °C durchgeführt. Anschließend wird heruntergekühlt und der Druck langsam abgelassen. Es werden 0,2 ml Isooctan als interner Standard zugesetzt. Umsatz und Ausbeute werden mittels GC und GC MS Analytik bestimmt.

Reaktionsbedingungen für Ligand **1**: Ein 25 ml-Schlenkgefäß wird mit einer Stammlösung aus [Pd(acac)₂] (12,2 mg, 0,04 mol), **1** (69,8 mg, 0,16 mmol), PTSA (114 mg, 0,6 mmol) und MeOH (25 ml) unter Argon befüllt. Ein 4 ml Glasvial versehen mit einem Magnetrührer, wird mit 2 mmol Olefin befüllt. Hierzu werden 1,25 ml der vorher hergestellten Stammlösung mittels einer Spritze zugesetzt. Dieses Vial wird auf einer Metallplatte in einem 300 ml-Parr-Autoklaven unter Argon platziert. Der Autoklav wird dreimal mit CO gespült und anschließend werden 40 bar CO aufgepreßt. Dann wird unter Magnetrührung die Reaktion 20 h bei 120 °C durchgeführt. Anschließend wird heruntergekühlt und der Druck langsam abgelassen. Es werden 0,2 ml Isooctan als interner Standard zugesetzt. Umsatz und Ausbeute werden mittels GC und GC MS Analytik bestimmt.

Für die GC Analyse wird in beiden Fällen ein Chromatograph der Firma Agilent GC Agilent 7890A mit einer 30 m HP Säule verwendet. Temperaturprofil: 35 °C, 10 min; 10 °C/min zu 285 °C, 285°C 5 min; das Injektionsvolumen beträgt 1 µl mit einem Split von 50:1.

Die Ergebnisse sind in den nachfolgenden beiden Tabellen dargestellt.

**Tabelle 3: Substratscreening mit den Liganden 3 (Vergleichsbeispiel) und 1 (erfindungsgemäßer Ligand)**

| Substrat | Ligand | Esterausbeute [%] (n/iso) |
|---|---|---|
| 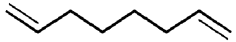 | **3** | 10 |
| Rt: 24,232-25,903 | **1*** | 94 (n,n/n,iso/iso,iso = 47/35/18) |
| 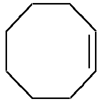 | **3** | 19 |
| | **1*** | 89 |
| Rt: 22,285 | | |
| 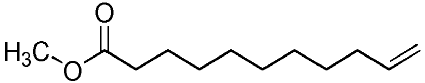 | **3** | 30 (92/8) |
| | **1*** | 96 (72/28) |
| Rt: 26,7-27,838 | | |
| 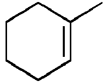 | **3** | 0 |
| | **1*** | 95^{a} (19/81) |
| Rt: 19,252-20,245 | | |
| 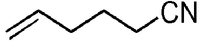 | **3** | 0 |
| Rt: iso:21,483, n: 22,327 | **1*** | 65 (84/16) |
| 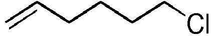 | **3** | 8 |
| Rt: iso:21,22, n: 222,062 | **1*** | 91 (70/30) |
| 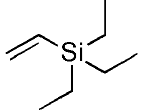 | **3** | 34 (100/0) |
| | **1*** | 88 (100/0) |
| Rt: 22,212 | | |
| 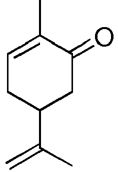 | **3** | 25 (100/0) |
| | **1*** | 97 (100/0) |
| Rt : 26,113 | | |
| 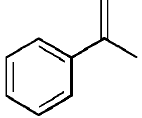 | **3** | 60 (100/0) |
| | **1*** | 95 (100/0) |
| Rt: 22,279 | | |
| 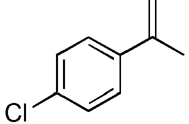 | **3** | 28 (100/0) |
| | **1*** | 97 (100/0) |
| Rt:24,477 | | |
| 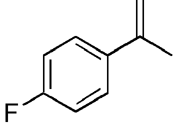 | **3** | 23 (100/0) |
| | **1*** | 96 (100/0) |
| Rt: 22,334 | | |
| 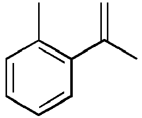 | **3** | 35 (100/0) |
| | **1*** | 96 (100/0) |
| Rt: 23,328 | | |
| 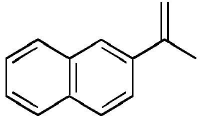 | **3** | 30 (100/0) |
| | **1*** | 97 (100/0) |
| Rt: 28,013 | | |
| 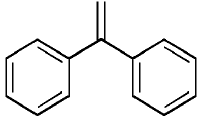 | **3** | 5 (100/0) |
| | **1*** | 99 (100/0) |
| RT: 27,883 | | |
| 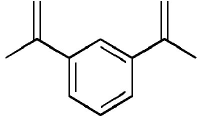 | **3** | 10 (100/0) |
| | **1*** | 99 (100/0) |
| Rt: 28,261 | | |
| 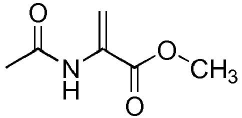 | **3** | 0 |
| | **1*** | 100 (100/0) |
| Rt : 23,814 | | |
| 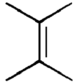 | **3** | 0 |
| | **1*** | 98 |
| Rt: 17,269 | | |

| | | |
|---|---|---|
| ^{a} GC Ausbeute. Rt: Retentionszeit in Minuten; * erfindungsgemäßer Ligand | | |

**Tabelle 4: Weitere Substrate im Screening mit den Liganden 3 (Vergleichsbeispiel) und 1 (erfindungsgemäßer Ligand) im direkten Vergleich**

| Substrat | Ligand | Esterausbeute (n/iso) [%] |
|---|---|---|
| 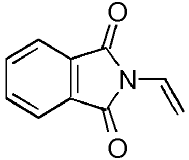 | **3** | 90 |
| | **1*** | 100 |
| Rt: 28,321 | | |
| 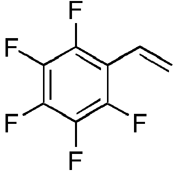 | **3** | 100 |
| | **1*** | 100 |
| Rt:21 ,038 | | |
| 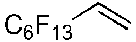 | **3** | 100 |
| Rt: 17.822 | **1*** | 100 |
| 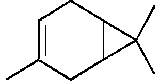 | **3** | 0 |
| | **1*** | 40 |
| Rt: 26.978-27.824 | | |
| 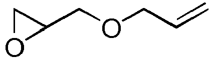 | **3** | 100 |
| | **1*** | 100 |
| Rt: 18.514 | | |
| 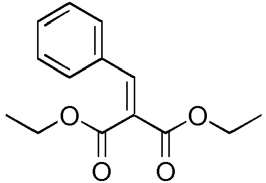 | **3** | 100 |
| | **1*** | 100 |
| Rt: 27.32 | | |
| 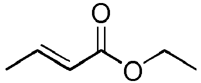 | **3** | 100 |
| | **1*** | 100 |

| | | |
|---|---|---|
| Rt: Retentionszeit in Minuten * erfindungsgemäßer Ligand | | |

Wie die Ergebnisse zeigen, können mit dem erfindungsgemäßen Verfahren eine Vielzahl unterschiedlicher ethylenisch ungesättigter Verbindungen umgesetzt werden. Dabei zeigt der erfindungsgemäße Ligand **1** in den meisten Fällen eine bessere Ausbeute an Ester, einen geringeren Isomerisierungsgrad, weniger Nebenproduktbildung und eine bessere n/iso-Selektivität als der Vergleichsligand **3.**

## Patentansprüche

1. Verbindung gemäß Formel (**I**) 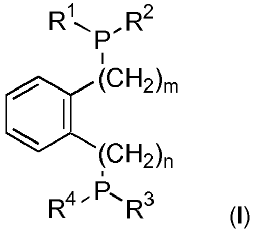 wobei
m und n unabhängig voneinander jeweils 0 oder 1 sind;
die Reste R¹ und R³ jeweils für einen -(C₃-C₂₀)-Heteroarylrest stehen;
und R² und R⁴ jeweils unabhängig voneinander ausgewählt sind aus -(C₁-C₁₂)-Alkyl und
R¹, R², R³, R⁴, falls diese für -(C₁-C₁₂)-Alkyl oder -(C₃-C₂₀)-Heteroaryl stehen, jeweils unabhängig voneinander mit einem oder mehreren Substituenten ausgewählt aus
-(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl,-(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

2. Verbindung nach Anspruch 1,
gemäß einer der Formeln (**II**) und (**III**)

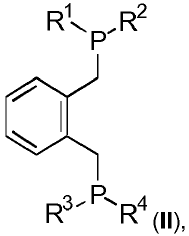

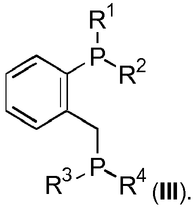

3. Verbindung nach einem der Ansprüche 1 bis 2,
wobei R¹, R³ jeweils unabhängig voneinander ausgewählt sind aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

4. Verbindung nach einem der Ansprüche 1 bis 3,
gemäß einer der Formeln (**1**) und (**18**)

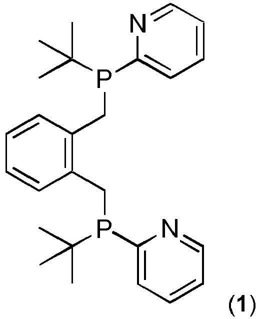

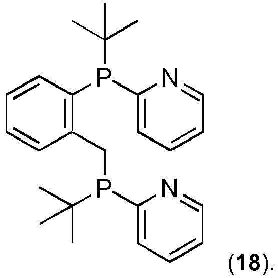

5. Komplex umfassend Pd und eine Verbindung nach einem der Ansprüche 1 bis 4.

6. Verfahren umfassend die Verfahrensschritte:
a) Vorlegen einer ethylenisch ungesättigten Verbindung;
b) Zugabe einer Verbindung nach einem der Ansprüche 1 bis 4 und einer Verbindung, welche Pd umfasst,
oder Zugabe eines Komplexes nach Anspruch 5;
c) Zugabe eines Alkohols;
d) Zuführen von CO;
e) Erwärmen des Reaktionsgemisches, wobei die ethylenisch ungesättigte Verbindung zu einem Ester umgesetzt wird.

7. Verfahren nach Anspruch 6,
wobei die ethylenisch ungesättigte Verbindung 2 bis 30 Kohlenstoffatom umfasst und optional eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten aufweist.

8. Verfahren nach einem der Ansprüche 6 bis 7,
wobei die ethylenisch ungesättigte Verbindung ausgewählt ist aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, di-n-Buten, oder Mischungen davon.

9. Verfahren nach einem der Ansprüche 6 bis 8,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

10. Verfahren nach einem der Ansprüche 6 bis 9,
wobei der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

11. Verwendung einer Verbindung gemäß einem der Ansprüche 1 bis 4 oder eines Komplexes gemäß Anspruch 5 zur Katalyse einer Alkoxycarbonylierungsreaktion.

## Claims

1. Compound of formula (**I**) 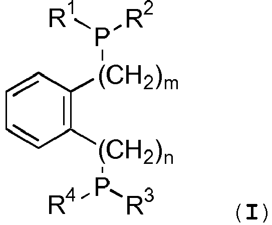 where
m and n are each independently 0 or 1;
the R¹ and R³ radicals are each a -(C₃-C₂₀)-heteroaryl radical;
and R² and R⁴ are each independently selected from - (C₁-C₁₂)-alkyl
and
R¹, R², R³, R⁴, if they are -(C₁-C₁₂)-alkyl or -(C₃-C₂₀)-heteroaryl,
may each independently be substituted by one or more substituents selected from -(C₁-C₁₂)-alkyl, -(C₃-C₁₂)-cycloalkyl, -(C₃-C₁₂)-heterocycloalkyl, -O-(C₁-C₁₂)-alkyl, -O-(C₁-C₁₂)-alkyl-(C₆-C₂₀)-aryl, -O-(C₃-C₁₂) -cycloalkyl, -S-(C₁-C₁₂)-alkyl, -S-(C₃-C₁₂) -cycloalkyl, -COO-(C₁-C₁₂)-alkyl, -COO-(C₃-C₁₂) -cycloalkyl, -CONH-(C₁-C₁₂)-alkyl, -CONH-(C₃-C₁₂)-cycloalkyl, -CO-(C₁-C₁₂)-alkyl, -CO-(C₃C₁₂)-cycloalkyl, -N-[(C₁-C₁₂)-alkyl]₂, -(C₆-C₂₀)-aryl, -(C₆-C₂₀)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -OH, -SO₃H, -NH₂, halogen.

2. Compound according to Claim 1,
of one of the formulae (**II**) and (**III**)

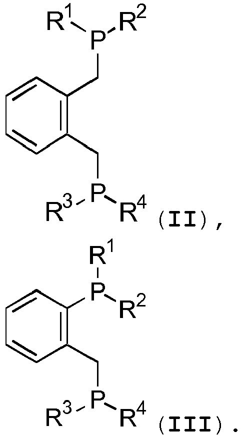

3. Compound according to either of Claims 1 and 2, where R¹, R³ are each independently selected from furyl, thienyl, pyrrolyl, oxazolyl, isoxazolyl, thiazolyl, isothiazolyl, imidazolyl, pyrazolyl, furazanyl, tetrazolyl, pyridyl, pyridazinyl, pyrimidyl, pyrazinyl, benzofuranyl, indolyl, isoindolyl, benzimidazolyl, quinolyl, isoquinolyl.

4. Compound according to any of Claims 1 to 3,
of one of the formulae (**1**) and (**18**)

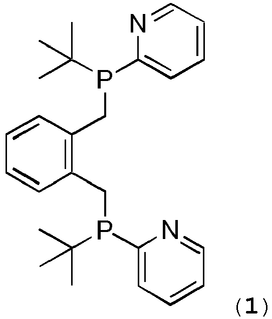

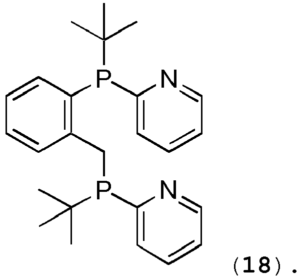

5. Complex comprising Pd and a compound according to any of Claims 1 to 4.

6. Process comprising the following process steps:
a) initially charging an ethylenically unsaturated compound;
b) adding a compound according to any of Claims 1 to 4 and a compound comprising Pd,
or adding a complex according to Claim 5;
c) adding an alcohol;
d) feeding in CO;
e) heating the reaction mixture, with conversion of the ethylenically unsaturated compound to an ester.

7. Process according to Claim 6,
wherein the ethylenically unsaturated compound comprises 2 to 30 carbon atoms and optionally one or more functional groups selected from carboxyl, thiocarboxyl, sulpho, sulphinyl, carboxylic anhydride, imide, carboxylic ester, sulphonic ester, carbamoyl, sulphamoyl, cyano, carbonyl, carbonothioyl, hydroxyl, sulphhydryl, amino, ether, thioether, aryl, heteroaryl or silyl groups and/or halogen substituents.

8. Process according to either of Claims 6 and 7,
wherein the ethylenically unsaturated compound is selected from ethene, propene, 1-butene, *cis*- and/or *trans*-2-butene, isobutene, 1,3-butadiene, 1-pentene, *cis*- and/or *trans-*2-pentene*,* 2-methyl-1-butene, 3-methyl-1-butene, 2-methyl-2-butene, hexene, tetramethylethylene, heptene, 1-octene, 2-octene, di-n-butene, and mixtures thereof.

9. Process according to any of Claims 6 to 8,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

10. Process according to any of Claims 6 to 9,
wherein the alcohol in process step c) is selected from methanol, ethanol, 1-propanol, 1-butanol, 1-pentanol, 1-hexanol, 2-propanol, tert-butanol, 3-pentanol, cyclohexanol, phenol, and mixtures thereof.

11. Use of a compound according to any of Claims 1 to 4 or of a complex according to Claim 5 for catalysis of an alkoxycarbonylation reaction.

## Revendications

1. Composé selon la formule (I) 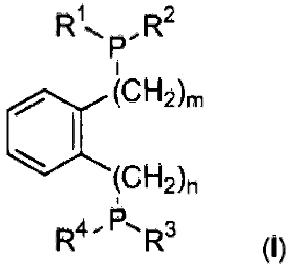 dans laquelle
m et n représentent chacun indépendamment l'un de l'autre 0 ou 1 ;
les radicaux R¹ et R³ représentent chacun un radical hétéroaryle en (C₃-C₂₀);
et R² et R⁴ sont chacun choisis indépendamment l'un de l'autre parmi alkyle en (C₁-C₁₂), et
R¹, R², R³, R⁴, si ceux-ci représentent alkyle en (C₁-C₁₂) ou hétéroaryle en (C₃-C₂₀), peuvent chacun indépendamment les uns des autres être substitués avec un ou plusieurs substituants choisis parmi :
alkyle en (C₁-C₁₂), cycloalkyle en (C₃-C₁₂), hétérocycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂), -CONH-alkyle en (C₁-C₁₂), -CONH-cycloalkyle en (C₃-C₁₂), -CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀)-O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀)-O-alkyle en (C₁-C₁₂), -COOH, -OH, -SO₃H, -NH₂, halogène.

2. Composé selon la revendication 1, selon l'une quelconque des formules (II) et (III)

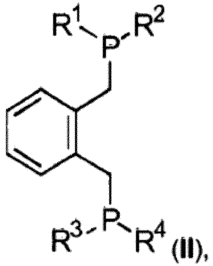

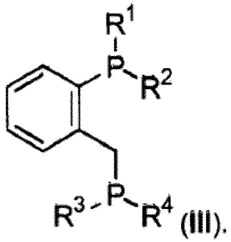

3. Composé selon l'une quelconque des revendications 1 à 2, dans lequel R¹, R³ sont chacun choisis indépendamment l'un de l'autre parmi furyle, thiényle, pyrrolyle, oxazolyle, isoxazolyle, thiazolyle, isothiazolyle, imidazolyle, pyrazolyle, furazanyle, tétrazolyle, pyridyle, pyridazinyle, pyrimidyle, pyrazinyle, benzofuranyle, indolyle, isoindolyle, benzimidazolyle, quinolyle, isoquinolyle.

4. Composé selon l'une quelconque des revendications 1 à 3, selon l'une quelconque des formules (1) et (18)

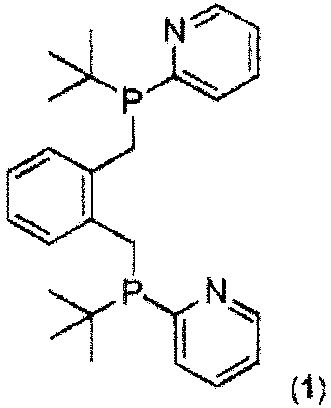

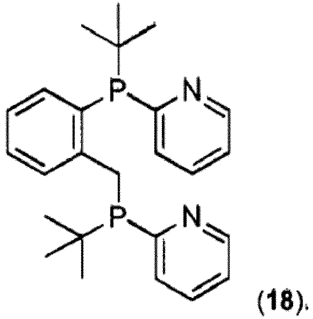

5. Complexe comprenant Pd et un composé selon l'une quelconque des revendications 1 à 4.

6. Procédé comprenant les étapes de procédé suivantes :
a) le chargement d'un composé éthyléniquement insaturé ;
b) l'ajout d'un composé selon l'une quelconque des revendications 1 à 4 et d'un composé qui comprend Pd,
ou l'ajout d'un complexe selon la revendication 5 ;
c) l'ajout d'un alcool ;
d) l'introduction de CO ;
e) le chauffage du mélange réactionnel, le composé éthyléniquement insaturé étant transformé en un ester.

7. Procédé selon la revendication 6, dans lequel le composé éthyléniquement insaturé comprend 2 à 30 atomes de carbone et comprend éventuellement un ou plusieurs groupes fonctionnels choisis parmi les groupes carboxyle, thiocarboxyle, sulfo, sulfinyle, anhydride d'acide carboxylique, imide, ester d'acide carboxylique, ester d'acide sulfonique, carbamoyle, sulfamoyle, cyano, carbonyle, carbonothioyle, hydroxyle, sulfhydryle, amino, éther, thioéther, aryle, hétéroaryle ou silyle et/ou les substituants halogène.

8. Procédé selon l'une quelconque des revendications 6 à 7, dans lequel le composé éthyléniquement insaturé est choisi parmi l'éthène, le propène, le 1-butène, le *cis-* et/ou le *trans*-2-butène, l'iso-butène, le 1,3-butadiène, le 1-pentène, le *cis-* et/ou le *trans*-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

9. Procédé selon l'une quelconque des revendications 6 à 8, dans lequel le composé qui comprend Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium(cinnamyle).

10. Procédé selon l'une quelconque des revendications 6 à 9, dans lequel l'alcool à l'étape de procédé c) est choisi parmi le méthanol, l'éthanol, le 1-propanol, le 1-butanol, le 1-pentanol, le 1-hexanol, le 2-propanol, le *tert*-butanol, le 3-pentanol, le cyclohexanol, le phénol ou leurs mélanges.

11. Utilisation d'un composé selon l'une quelconque des revendications 1 à 4 ou d'un complexe selon la revendication 5 pour la catalyse d'une réaction d'alcoxycarbonylation.
